# EUROPEAN PATENT APPLICATION

(11) **EP 4 681 709 A1**
(43) Date of publication of application: **21.01.2026**
(21) Application number: 24188695.1
(22) Date of filing: 15.07.2024
(51) Int. Cl.: A61K 31/198, A61K 31/519, A61K 31/714, A61K 33/30, A61K 36/16, A61K 36/537, A61K 36/77, A61K 36/87, A61P 25/00, A61P 25/24, A23L 33/00

(54) **MENTAL WELLBEING COMPOSITIONS**

(71) Applicant: FoodFor Labs AG, 8001 Zürich (CH)
(72) Inventor: Cramer, Teresa, Cambridge, 02139 (US); Kuenburg, Franziska, 8001 Zürich (CH); Thurn-Taxis, Raphael, 8005 Zürich (CH)
(74) Representative: Troesch Scheidegger Werner AG

(57) **Abstract**

Compositions for improving mental wellbeing and associated uses.

## Description

### TECHNICAL FIELD OF THE INVENTION

The current invention relates to compositions for improving mental wellbeing, as well as to associated uses thereof.

### DESCRIPTION OF THE RELATED ART

Mental discomforts are widespread among different populations. Known and standard approaches to improving mental wellbeing include the administration of pharmaceutical agents with potential side effects and the necessity for coherent medical supervision. Known alternatives to pharmaceuticals include nutrition-based approaches and nutrition supplements to improve mental wellbeing. However, current supplements typically fall short on being convenient (unappealing pill format), and are not tailored to specific mental needs. Importantly, known supplements more often offer temporary relief of specific symptoms rather than providing a comprehensive solution that targets the neurobiological/cognitive processes of psychological disfunction at the root of mental health challenges.

### SUMMARY OF THE INVENTION

It is a task of current invention to provide compositions for improving mental wellbeing.

This task is solved by a composition with the features of claims 1 to 4. Further embodiments of the composition, as well as a use of the composition are defined by the features of further claims.

In a first aspect, the present invention is directed to a composition for oral use comprising:
- Vitamin B12;
- Vitamin B9;
- Zinc;
- N-Acetyl-L-Tyrosine, optionally as L-Tyrosine;
- Phenylalanine;
- Green tea extract, optionally a water extract, optionally of the arial parts of the green tea, optionally comprising 40 weight% to 70 weight%, optionally 44 to 66 weight% or about 60 weight% L-Theanine;
- Guarana extract, optionally a water-ethanol extract, e.g. 60 vol% to 80 vol% ethanol in water, optionally of the seeds of guarana, optionally comprising 15 weight% to 70 weight%, optionally about 30 to 50 weight% caffein;
- Ginko biloba extract, optionally a water-ethanol extract, e.g. 60 vol% to 80 vol% ethanol in water, optionally at an extract ratio of 50/1 to 52/1, optionally of the ariel parts of ginkgo biloba; and
- Sage leaf extract, optionally a water extract, optionally at an extract ratio of 3/1 to 4/1.

As used herein for all aspects and embodiments, the extract ratio refers to the ratio of the mass of dried herbal/plant material to the mass of the resulting extract. For example, the extracts for use in the present invention are standardized extracts as known to the skilled person and are, e.g., prepared, i.e. extracted, according to standards utilized in extraction techniques. For example, these standards include the European Pharmacopoeia monograph 01/2008:20817; 02/2008:20816; 04/2019:20934, 01/2008:20416, 07/2016:20203, 07/2010:20612, 01/2008:1523 and 01/2014:20631 for quality control and standardization and utilize methods such granulometry, titration, HPLC (high performance liquid chromatography), UV-VIS (ultraviolet-visible spectrophotometry), TLC (Thin layer chromatography), GC-MS (Gas chromatography-mass spectrometry), LC-MS (Liquid chromatography-mass spectrometry) and ICP-OES (Inductively coupled plasma optical emission spectrometry) for determining the quantity, purity, and identification of active ingredients and impurities in plant products.

For example, the first composition can be used to improve normal psychological function, by improving focus, stimulating mental alertness, supporting memory performance and/or relieving fatigue.

In a second aspect, the present invention is directed to a composition for oral use comprising:
- Vitamin B3;
- Vitamin B1;
- Manganese;
- L-Glutamine;
- L-Carnitine;
- Caffeine; and
- Rhodiola Rosea extract, optionally a water-ethanol extract, e.g. 65 vol% to 75 vol% ethanol in water, optionally at an extract ratio of 1/1 to 2/1, optionally of the roots of Rhodiola Rosea.

For example, the second composition can be used to improve normal psychological functioning by reducing tiredness and fatigue, relieving sensations of weakness, improving an energy-yielding metabolism and protecting cells from oxidative stress.

In a third aspect, the present invention is directed to a composition for oral use comprising:
- Vitamin B6;
- Vitamin D3;
- L-Tryptophan;
- Inositol;
- Taurine;
- Panax Ginseng extract, optionally a water-ethanol extract, e.g. 20 vol% to 40 vol% ethanol in water, e.g. about 30 vol% ethanol in water, optionally at an extract ratio of 8/1 to 10/1, optionally of the roots of Panax Ginseng, optionally comprising 10 weight% to 25 weight%, optionally 12 weight% to 16 weight% ginsenosides;
- Yerba Mate extract, optionally a water extract, optionally at an extract ratio of 1/1 to 5/1, optionally of the ariel parts of Yerba Mate, optionally comprising 15 weight% to 30 weight%, optionally about 20 weight% caffein; and
- Cocoa bean extract, optionally a water extract, optionally at an extract ratio of 7/1 to 9/1, optionally about 8/1, optionally of the seeds of Cocoa bean, optionally comprising 5 weight% to 10 weight%, optionally about 7 weight% theobromine.

For example, the third composition can be used to improve normal psychological functioning by improving emotional well-being, supporting positive mood, enhancing sensations of vitality and increasing relaxation.

In fourth aspect, the present invention is directed to a composition for oral use comprising:
- Magnesium;
- L-Glutamic acid;
- L-Arginine;
- Glycine;
- Ashwagandha root extract, optionally a water extract, optionally at an extract ratio of 10/1 to 25/1, optionally about 20/1, optionally comprising 1.8 weight% to 3.5 weight%, optionally about 2.5 weight! withanolides;
- Valerian root extract, optionally a water extract, optionally at an extract ratio of 9/1 to 12/1, optionally about 11/1, optionally comprising 0.3 weight% to 1.6 weight%, optionally about 0.8 weight% valerian acids;
- Lemon balm extract, optionally a water-ethanol extract, e.g. 10 vol% to 50 vol% or about 30 vol% ethanol in water, optionally at an extract ratio of 3/1 to 7/1, optionally about 5/1, optionally of the arial parts of Lemon balm; and
- Hops extract, optionally a water-ethanol extract, e.g. 25 vol% to 30 vol% ethanol in water, optionally at an extract ratio of 4/1 to 6/1, optionally of the arial parts of Hops.

Valerian acids, as used herein, include valeric acid, isovaleric acid, valerenic acid, hydroxyvalerenic acid and acetoxyvalerenic acid.

For example, the fourth composition can be used to improve normal psychological functioning by improving calmness, relieving mental and nervous tension and promoting sleep onset / relieving difficulty in falling asleep.

It was surprisingly found that the four compositions detailed above share that they are all effective in improving different aspects of normal psychological function and thereby different aspects of mental wellbeing.

Without wishing to be bound by theory, it is believed that the compositions positively influence the function of different neuronal pathways and their associated neurotransmitters, which underlie these varying aspects of psychological functioning. It is believed that by targeting multiple aspects of neuronal pathway function (production, transduction and release/response of associated neurotransmitters) by using a diverse and highly specific group of ingredients (vitamins, minerals, amino acids and phytochemicals), a more effective improvement of pathway function and mental well-being is achieved compared to the administration of individual ingredients. In other words, it is believed to be the combination of the ingredients that achieves the effect of improving mental wellbeing, which goes beyond the individual effect of each component.

The features of the above-mentioned embodiments of the compositions can be used in any combination within the scope of the claims, provided they do not contradict each other.

In an embodiment of the compositions according to the present invention which may be combined with any of the embodiments and aspects preaddressed or still to be addressed unless in contradiction, the composition, in particular a daily dose and/or 60 mL thereof, comprises:
- 1 µg to 6 pg, optionally 1.5 µg to 4 pg, optionally about 2.5 µg Vitamin B12;
- 100 µg to 500 pg, optionally 120 µg to 300 pg, optionally about 200 µg Vitamin B9;
- 1 mg to 10 mg, optionally 2 to 7 mg, optionally about 5 mg Zinc;
- 200 mg to 100 mg, optionally 300 to 700 mg, optionally about 500 mg N-Acetyl-L-Tyrosine;
- 100 mg to 500 mg, optionally 150 to 350 mg, optionally about 250 mg Phenylalanine;
- 200 mg to 1000 mg, optionally 250 to 800 mg, optionally about 330 mg green tea extract;
- 80 mg to 600 mg, optionally 150 to 300 mg, optionally about 200 mg Guarana extract;
- 50 mg to 350 mg, optionally 90 to 180 mg, optionally about 120 mg Ginko biloba extract; and
- 50 mg to 250 mg, optionally 60 mg to 150 mg, optionally about 100 mg Sage leaf extract.

As defined herein, the reference to 60 mL means that a total volume of 60 mL comprises the components as indicated, wherein the volume is typically a liquid, in particular an aqueous solution. It is noted that any multiple or fracture of 60 mL is within the scope of the present invention, provided that the amounts of the ingredients are adjusted by the same factor. The 60 mL volume is, for example, generally considered a daily dose of the composition which can be safely and effectively consumed once a day to achieve an improvement in mental wellbeing while each component of the composition is within the daily dose limits set forth by health authorities, e.g., by the European Food Safety Authority (EFSA). In other words, the definition "60 mL" can be replaced by the term "a daily dose of the composition" or a "a dosage suitable for daily administration".

In an embodiment of the compositions according to the present invention which may be combined with any of the embodiments and aspects preaddressed or still to be addressed unless in contradiction, the composition, in particular a daily dose and/or 60 mL thereof, comprises:
- 5 mg to 35 mg, optionally 8 mg to 25 mg, optionally about 15 or 16 mg Vitamin B3;
- 0.5 mg to 3 mg, optionally 0.8 mg to 2 mg, optionally about 1.0 mg or 1.1 mg Vitamin B1;
- 0.3 mg to 5 mg, optionally 1 mg to 3 mg, optionally about 2 mg Manganese;
- 100 mg to 500 mg, optionally 150 to 350 mg, optionally about 250 mg L-Glutamine;
- 100 mg to 500 mg, optionally 150 to 350 mg, optionally about 250 mg L-Carnitine;
- 30 mg to 200 mg, optionally 50 mg to 130 mg, optionally about 90 mg caffeine; and
- 25 mg to 150 mg, optionally 40 mg to 85 mg, optionally about 65 mg Rhodiola Rosea extract.

In an embodiment of the compositions according to the present invention which may be combined with any of the embodiments and aspects preaddressed or still to be addressed unless in contradiction, the composition, in particular a daily dose and/or 60 mL thereof, comprises:
- 0.5 mg to 5 mg, optionally 1 mg to 2.5 mg, optionally about 1.5 mg or 1.4 mg Vitamin B6;
- 1 µg to 50 pg, optionally 1 µg to 10 pg, optionally 2 µg to 7 pg, optionally about 5 µg Vitamin D3;
- 150 mg to 850 mg, optionally 300 to 500 mg, optionally about 400 mg L-Tryptophan;
- 200 mg to 100 mg, optionally 300 to 700 mg, optionally about 500 mg Inositol;
- 200 mg to 100 mg, optionally 300 to 700 mg, optionally about 500 mg Taurine;
- 10 mg to 100 mg, optionally 20 mg to 60 mg, optionally about 40 mg Panax Ginseng extract;
- 100 mg to 1000 mg, optionally 200 mg to 600 mg, optionally about 400 mg Yerba Mate extract; and
- 50 mg to 270 mg, optionally 80 to 150 mg, optionally about 100 mg Cocoa bean extract.

In an embodiment of the compositions according to the present invention which may be combined with any of the embodiments and aspects preaddressed or still to be addressed unless in contradiction, the composition, in particular a daily dose and/or 60 mL thereof, comprises:
- 80 mg to 400 mg, optionally 100 to 250 mg, optionally about 190 mg or 188 mg Magnesium;
- 50 mg to 450 mg, optionally 100 to 300 mg, optionally about 200 mg L-Glutamic acid;
- 150 mg to 850 mg, optionally 300 to 500 mg, optionally about 400 mg L-Arginine;
- 150 mg to 850 mg, optionally 300 to 500 mg, optionally about 400 mg Glycine;
- 150 mg to 250 mg, optionally 180 to 220 mg, optionally about 200 mg Ashwagandha root extract;
- 15 mg to 60 mg, optionally 25 to 35 mg, optionally about 30 mg or 28 mg Valerian root extract;
- 45 mg to 180 mg, optionally 60 mg to 120 mg, optionally about 90 mg Lemon balm extract; and
- 5 mg to 50 mg, optionally about 10 mg to 30 mg, optionally about 20 mg Hops extract.

In an embodiment of the compositions according to the present invention which may be combined with any of the embodiments and aspects preaddressed or still to be addressed unless in contradiction, the composition is a food, food additive, drink, or drink additive.

For example, the composition may either be pre-dissolved as a drink, or it may be in the form of a powder to be dissolved in a drink, in particular to be dissolved to a volume of 60 mL or a multiple or fraction thereof, while adjusting the amounts of the components accordingly.

In an embodiment of the compositions according to the present invention which may be combined with any of the embodiments and aspects preaddressed or still to be addressed unless in contradiction, the composition further comprises at least one of a flavoring, optionally a natural flavoring or a sweetener, a colorant, carbon dioxide, a stabilizer, optionally guar gum, a preservative, optionally citric acid and a combination thereof.

Guar gum can be used in the composition as a stabilizer, an emulsifier, a thickener, and as a soluble dietary fiber.

In an embodiment of the compositions according to the present invention which may be combined with any of the embodiments and aspects preaddressed or still to be addressed unless in contradiction, the composition is a dry product for dissolution, optionally for dissolution in water. As noted above, the composition may, for example, be dissolved to a volume of 60 mL or a multiple or fraction thereof, while adjusting the amounts of the components accordingly.

In an embodiment of the compositions according to the present invention which may be combined with any of the embodiments and aspects preaddressed or still to be addressed unless in contradiction, the composition is a water-based solution, optionally a water-based solution that is packaged in a container, optionally a solution with a volume of 50 to 150 mL, optionally of about 60 mL.

In an embodiment of the compositions according to the present invention which may be combined with any of the embodiments and aspects preaddressed or still to be addressed unless in contradiction, the composition is a pharmaceutical composition.

The pharmaceutical composition may be in any suitable dosage form such as a liquid, a powder, a capsule or a tablet.

In an aspect which may be combined with any of the aspects and embodiments preaddressed or still to be addressed unless in contradiction, the invention is directed to the composition as defined herein for use as a medicament, optionally as a nutritional supplement.

In an aspect which may be combined with any of the aspects and embodiments preaddressed or still to be addressed unless in contradiction, the invention is directed to the composition as defined herein for use in the therapy of mental health problems and/or for use in the improvement of mental wellbeing, optionally for use in the treatment of depression, stress, fatigue, insomnia and/or mood fluctuations.

The term "mental wellbeing" as used herein, in particular in the context of improving mental wellbeing, refers to, a state of comfort, health, or happiness that integrates various aspects of brain function (e.g. neuronal activity and neurotransmitter balance), cognitive processes (e.g. thought patterns, memory, attention, and problem-solving) and psychological functioning (e.g. emotional regulation and behavior).

Improving mental wellbeing can further include, e.g., the improvement of mental and/or psychological illnesses or mental health problems, in particular the improvement of depression, depressive mood, sleep disorders, mood disorder, fatigue syndromes, attention deficit problem or disorder, generalized anxiety disorder, panic disorder, obsessive-compulsive disorder, post-traumatic stress disorder, social anxiety disorder, phobias, and dementia.

### BRIEF DESCRIPTION OF THE DRAWINGS

Embodiments of the current invention are described in more detail in the following with reference to the figures. These are for illustrative purposes only and are not to be construed as limiting. It shows
Fig. 1 results of the self-report questionnaire;
Fig. 2 results of the self-report questionnaire;
Fig. 3 results of the self-report questionnaire; and
Fig. 4 results of the self-report questionnaire.

### DETAILED DESCRIPTION OF THE INVENTION

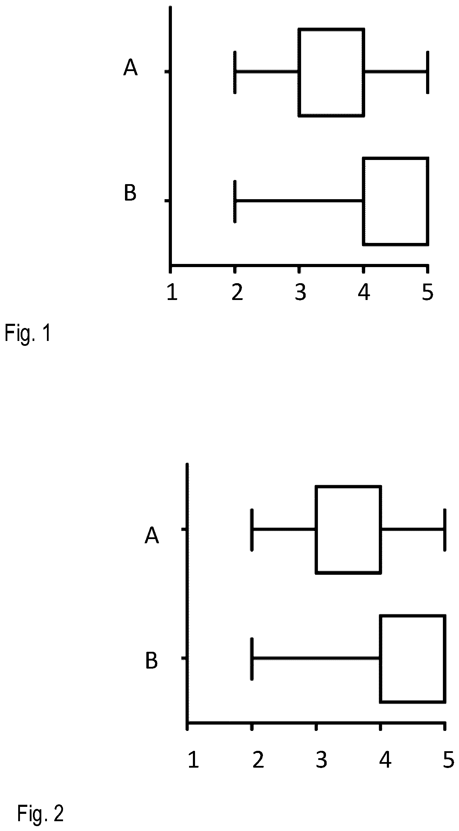

**Figure 1** shows the plots of the results of the self-report questionnaire of participants after consumption of Composition 1 (Example 1.1.) obtained according to Example 2. The y axis refers to statements (A) and (B), the x axis refers to how the statements applied. The participants were asked whether statements (A) and (B) 6 to 12 hours (nocturnal sleep period ) after consumption of the Composition applied(1): not at all, (2) very slightly, (3) moderately, (4) quite a bit and (5) extremely. Statement (A) was: "I felt my overall sleep quality improved" and statement (B) was "I fell asleep faster than usual". It is apparent that the majority of the participants felt a positive effect of the Composition on their mental wellbeing in that the sleep quality was improved and that they could fall asleep faster than usual.

**Figure 2** shows the plots of the results of the self-report questionnaire of participants after consumption of Composition 2 (Example 1.2.) obtained according to Example 2. The y axis refers to statements (A) and (B), the x axis refers to how the statements applied. The participants were asked whether statements (A) and (B) 1 to 4 hours after consumption of the Composition applied (1): not at all, (2) very slightly, (3) moderately, (4) quite a bit and (5) extremely. Statement (A) was: " I was able to complete task more efficiently" and statement (B) was " I felt more concentrated and attentive". It is apparent that the majority of the participants felt a positive effect of the Composition on their mental wellbeing in that tasks could be completed more efficiently and that they felt more concentrated and attentive.

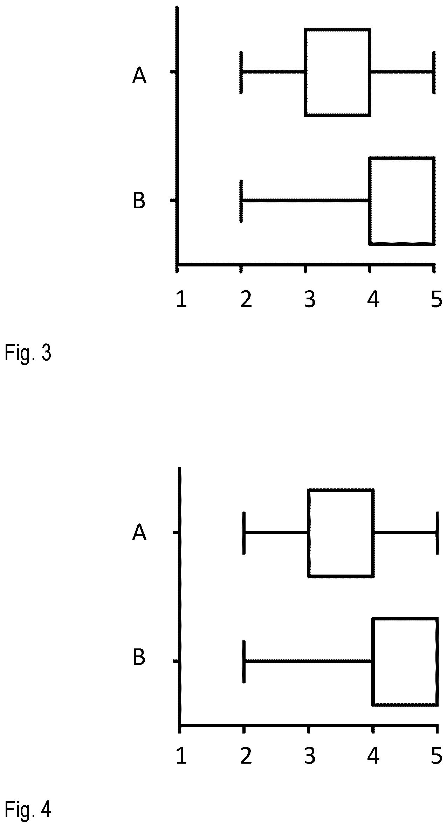

**Figure 3** shows the plots of the results of the self-report questionnaire of participants after consumption of Composition 3 (Example 1.3.) obtained according to Example 2. The y axis refers to statements (A) and (B), the x axis refers to how the statements applied. The participants were asked whether statements (A) and (B) 1 to 4 hours after consumption of the Composition applied (1): not at all, (2) very slightly, (3) moderately, (4) quite a bit and (5) extremely. Statement (A) was: "I felt more positive about my day's accomplishments" and statement (B) was " my overall mood was elevated". It is apparent that the majority of the participants felt a positive effect of the Composition on their mental wellbeing in that they were more positive about their accomplishments and that their mood was elevated.

**Figure 4** shows the plots of the results of the self-report questionnaire of participants after consumption of Composition 4 (Example 1.4.) obtained according to Example 2. The y axis refers to statements (A) and (B), the x axis refers to how the statements applied. The participants were asked whether statements (A) and (B) 1 to 4 hours after consumption of the Composition applied (1): not at all, (2) very slightly, (3) moderately, (4) quite a bit and (5) extremely. Statement (A) was: "I felt more positive about my day's accomplishments" and statement (B) was " my overall mood was elevated". It is apparent that the majority of the participants felt a positive effect of the Composition on their mental wellbeing in that they were more positive about their accomplishments and that their mood was elevated.

### Examples

### 1. Examples of compositions

### 1.1. Composition 1

The following composition was made. In an example, the composition was dissolved in water to a total volume of 60 mL. In an example, the composition is a daily dose.

| **Ingredient Name** | **Extraction Solvent** | **Part of Plant** | **Amount** |
|---|---|---|---|
| | | | |
| Vitamin B12 | - | - | 2.5 µg |
| Vitamin B9 (Folate) | - | - | 200 µg |
| Zinc | - | - | 5 mg |
| N-Acetyl-L-Tyrosine | - | - | 500 mg |
| Phenylalanine | - | - | 250 mg |
| NeuroTeyanin Green Tea extract | 100% Water | Arial Part | 330 mg* |
| Guarana extract | 30% Water / 70% Ethanol | Seeds | 200 mg** |
| Ginkgo biloba extract | 20-40 % Water/ 60-80% Ethanol | Ariel part | 120 mg |
| Sage leaf extract | 100% Water | Arial Part | 100 mg |

| | | | |
|---|---|---|---|
| *: for example, calculated to yield 198 mg L-Theanine **: for example, calculated to yield 66 mg caffeine | | | |

### 1.2. Composition 2

The following composition was made. In an example, the composition was dissolved in water to a total volume of 60 mL. In an example, the composition is a daily dose.

| **Ingredient Name** | **Extraction Solvent** | **Part of Plant** | **Amount** |
|---|---|---|---|
| | | | |
| Vitamine B3 (Niacin) | - | - | 16 mg |
| Vitamin B1 (Thiamine) | - | - | 1.1 mg |
| Manganese | - | - | 2 mg |
| L-Glutamine | - | - | 250 mg |
| L-carnitine | - | - | 250 mg |
| Caffeine | - | - | 90 mg |
| Rhodiola Rosea extract | 70% Ethanol / 30% Water | Root | 65 mg |

### 1.3. Composition 3

The following composition was made. In an example, the composition was dissolved in water to a total volume of 60 mL. In an example, the composition is a daily dose.

| **Ingredient Name** | **Extraction Solvent** | **Part of Plant** | **Amount** |
|---|---|---|---|
| Vitamin B6 | - | - | 1.4 mg |
| Vitamin D3 | - | - | 5 µg |
| L-Tryptophan | - | - | 400 mg |
| Inositol | - | - | 500 mg |
| Taurine | - | - | 500 mg |
| Panax Ginseng extract | Ethanol 30% / Water 70% | Root | 40 mg |
| Yerba Mate extract | 100% Water | Ariel part | 400 mg* |
| Cocoa bean extract | 100% Water | Seed | 100 mg |

| | | | |
|---|---|---|---|
| *: for example, calculated to yield 80 mg caffeine | | | |

### 1.4. Composition 4

The following composition was made. In an example, the composition was dissolved in water to a total volume of 60 mL. In an example, the composition is a daily dose.

| **Ingredient Name** | **Extraction Solvent** | **Part of Plant** | **Amount** |
|---|---|---|---|
| | | | |
| Magnesium | - | - | 187.5 mg |
| L-Glutamic acid | - | - | 200 mg |
| L-Arginine | - | - | 400 mg |
| Glycine | - | - | 400 mg |
| Ashwagandha root extract | 100% Water | Root | 200mg* |
| Valerian Root extract | 100% Water | Root | 30 mg |
| Lemon balm extract | Water70% / Ethanol 30% | Arial part | 90 mg |
| Hops extract | Ethanol 25-30% / Water 70-75% | Arial part | 20 mg |

| | | | |
|---|---|---|---|
| *: for example, calculated to yield 5 mg of withanolides | | | |

### 2. Examples of effects

To assess the effect of the compositions disclosed and claimed herein, 12 volunteers were provided with four different compositions and asked to (a) provide information on their lifestyle and base mental wellbeing, and (b) on the effects they experienced after consuming the compositions as outlined in the description of Figures 1 to 4. The questions were asked in the context of a written self-report questionnaire. The results of the self-report questionnaire are summarized and analyzed in Figures 1 to 4.

## Claims

1. A composition for oral use comprising:
- Vitamin B12;
- Vitamin B9;
- Zinc;
- N-Acetyl-L-Tyrosine;
- Phenylalanine;
- Green tea extract;
- Guarana extract;
- Ginko biloba extract; and
- Sage leaf extract.

2. A composition for oral use comprising:
- Vitamin B3;
- Vitamin B1;
- Manganese;
- L-Glutamine;
- L-Carnitine;
- Caffeine; and
- Rhodiola Rosea extract.

3. A composition for oral use comprising:
- Vitamin B6;
- Vitamin D3;
- L-Tryptophan;
- Inositol;
- Taurine;
- Panax Ginseng extract;
- Yerba Mate extract; and
- Cocoa bean extract.

4. A composition for oral use comprising:
- Magnesium;
- L-Glutamic acid;
- L-Arginine;
- Glycine;
- Ashwaganda root extract;
- Valerian root extract;
- Lemon balm extract; and
- Hops extract.

5. The composition according to claim 1, wherein the composition, optionally 60 mL of the composition, comprises:
- 1 µg to 6 pg, optionally 1.5 µg to 4 pg, optionally about 2.5 µg Vitamin B12;
- 100 µg to 500 pg, optionally 120 µg to 300 pg, optionally about 200 µg Vitamin B9;
- 1 mg to 10 mg, optionally 2 to 7 mg, optionally about 5 mg Zinc;
- 200 mg to 100 mg, optionally 300 to 700 mg, optionally about 500 mg N-Acetyl-L-Tyrosine;
- 100 mg to 500 mg, optionally 150 to 350 mg, optionally about 250 mg Phenylalanine;
- 200 mg to 1000 mg, optionally 250 to 800 mg, optionally about 330 mg green tea extract;
- 80 mg to 600 mg, optionally 150 to 300 mg, optionally about 200 mg Guarana extract;
- 50 mg to 350 mg, optionally 90 to 180 mg, optionally about 120 mg Ginko biloba extract; and
- 50 mg to 250 mg, optionally 60 mg to 150 mg, optionally about 100 mg Sage leaf extract.

6. The composition according to claim 2, wherein the composition, optionally 60 mL of the composition, comprises:
- 5 mg to 35 mg, optionally 8 mg to 25 mg, optionally about 15 or 16 mg Vitamin B3;
- 0.5 mg to 3 mg, optionally 0.8 mg to 2 mg, optionally about 1.0 mg or 1.1 mg Vitamin B1;
- 0.3 mg to 5 mg, optionally 1 mg to 3 mg, optionally about 2 mg Manganese;
- 100 mg to 500 mg, optionally 150 to 350 mg, optionally about 250 mg L-Glutamine;
- 100 mg to 500 mg, optionally 150 to 350 mg, optionally about 250 mg L-Carnitine;
- 30 mg to 200 mg, optionally 50 mg to 130 mg, optionally about 90 mg caffeine; and
- 25 mg to 150 mg, optionally 40 mg to 85 mg, optionally about 65 mg Rhodiola Rosea extract.

7. The composition according to claim 3, wherein the composition, optionally 60 mL of the composition, comprises:
- 0.5 mg to 5 mg, optionally 1 mg to 2.5 mg, optionally about 1.5 mg or 1.4 mg Vitamin B6;
- 1 µg to 50 pg, optionally 1 µg to 10 pg, optionally 2 µg to 7 pg, optionally about 5 µg Vitamin D3;
- 150 mg to 850 mg, optionally 300 to 500 mg, optionally about 400 mg L-Tryptophan;
- 200 mg to 100 mg, optionally 300 to 700 mg, optionally about 500 mg Inositol;
- 200 mg to 100 mg, optionally 300 to 700 mg, optionally about 500 mg Taurine;
- 10 mg to 100 mg, optionally 20 mg to 60 mg, optionally about 40 mg Panax Ginseng extract;
- 100 mg to 1000 mg, optionally 200 mg to 600 mg, optionally about 400 mg Yerba Mate extract; and
- 50 mg to 270 mg, optionally 80 to 150 mg, optionally about 100 mg Cocoa bean extract.

8. The composition according to claim 4, wherein the composition, optionally 60 mL of the composition, comprises:
- 80 mg to 400 mg, optionally 100 to 250 mg, optionally about 190 mg or 188 mg Magnesium;
- 50 mg to 450 mg, optionally 100 to 300 mg, optionally about 200 mg L-Glutamic acid;
- 150 mg to 850 mg, optionally 300 to 500 mg, optionally about 400 mg L-Arginine;
- 150 mg to 850 mg, optionally 300 to 500 mg, optionally about 400 mg Glycine;
- 100 mg to 300 mg, optionally 150 to 250 mg, optionally about 200 mg Ashwagandha root extract;
- 15 mg to 60 mg, optionally 25 to 35 mg, optionally about 30 mg or 28 mg Valerian root extract;
- 45 mg to 180 mg, optionally 60 mg to 120 mg, optionally about 90 mg Lemon balm extract; and
- 5 mg to 50 mg, optionally about 10 mg to 30 mg, optionally about 20 mg Hops extract.

9. The composition according to any of claims 1 to 8, wherein the composition is a food, food additive, drink, or drink additive.

10. The composition according to any of claims 1 to 9, wherein the composition further comprises at least one of a flavoring, optionally a natural flavoring or a sweetener, a colorant, carbon dioxide, a stabilizer, optionally guar gum, a preservative, optionally citric acid and a combination thereof.

11. The composition according to any of claims 1 to 10, wherein the composition is a dry product for dissolution, optionally for dissolution in water.

12. The composition according to any of claims 1 to 10, wherein the composition is a water-based solution, optionally a water-based solution that is packaged in a container, optionally a solution with a volume of 50 to 150 mL, optionally of about 60 mL.

13. The composition according to any of claims 1 to 12, wherein the composition is a pharmaceutical composition.

14. The composition according to any of claims 1 to 13 for use as a medicament.

15. The composition according to any of claims 1 to 14 for use in the improvement of mental wellbeing, optionally for use in the treatment of depression, stress, tiredness, and/or mood fluctuations.
